# EUROPEAN PATENT APPLICATION

(11) **EP 1 043 397 A1**
(43) Date of publication of application: **11.10.2000**
(21) Application number: 99949429.7
(22) Date of filing: 26.10.1999
(51) Int. Cl.: C12N 15/54, C12N 9/12, A61K 31/70, A61K 38/46

(54) **IDENTIFICATION OF NOVEL SUBSTRATE I-TRAF OF IKK-i KINASE**

(30) Priority: 26.10.1998 JP 30408598
(71) Applicant: Japan Science and Technology Corporation, Kawaguchi-shi, Saitama-ken 332-0012 (JP)
(72) Inventor: AKIRA, Shizuo, Minoo-shi, Osaka-fu 562-0031 (JP); SHIMADA, Takahiro, Takaishi-shi, Osaka-fu 592-0013 (JP)
(74) Representative: Cresswell, Thomas Anthony
(86) International application number: JP9905916
(87) International publication number: WO0024908

(57) **Abstract**

Novel IkB kinase IKK-i which is a novel serine/threonine kinase capable of activating a transcription factor NF-kB which inhibits the expression of various genes relating to immune response; a gene encoding the same; and medicinal compositions containg the same.

## Description

### Technical Field

The present invention relates to a novel IκB kinase, the gene for it and a pharmaceutical composition containing it. More particularly, the present invention pertains to a novel IκB kinase, IKK-i, which is a novel serine/threonine kinase capable of activating transcription factor NF-κ B which regulates expression of various genes involved in immune response and binds with I-TRAF to phosphorylate it.

### Background Art

Macrophages play an important role in biological defense mechanisms and are known to play major roles in functions such as phagocytosis. They are also involved in antigen presentation against bacterial infections and infiltration by malignant tumor. Further, macrophages are activated by lipopolysaccharide (LPS) and inflammatory cytokines, and they express various genes involved in the immune response. These include the major histocompatibility antigen, TNF-α (tumor necrosis factor-α), IL-1β (interleukin- 1β), IL-6 (interleukin-6) and MIP-1α/β (macrophage inflammatory protein-1α/β).

NF-κB is a transcription factor which regulates the expression of various genes involved in the immune response. NF-κB is known to be activated by LPS, TNF-α and IL-1β, and to regulate transcription of genes of TNF-α, IL-1β and Iκ B-α, which have important roles in the immune response.

In order to identify a novel gene involved in the immune response, the inventors have performed subtractive hybridization between a (+) group and a (-) group of LPS stimulation from the macrophage tumor strain, RAW 264.7. The gene obtained in this way, done #2F9, was demonstrated to be a novel gene having homology with IκB kinase-α,β (DiDonato, J.A., et al., Nature 1997, Aug. 7, 388 (6642), 548-554; Zandi, E. et al., Cell 1997, Oct. 17, 91(2), 243-252; Mercurio, F.et al., Science 1997, Oct. 31, 278(5339), 860-866; Woronicz, J.D., et al., Science 1997, Oct. 31, 278 (5339), 866-869; Regnier, C.H., et al., Cell 1997, Jul. 25, 90(2), 373-383). This gene was identified recently and is known to activate NF-κB. The inventors have given the name IKK-i (inducible-IκB kinase) to the protein coded by this novel gene.

### Disclosure of Invention

The present invention provides a novel IκB kinase, IKK-i, which is a novel serine/threonin kinase capable of activating transcription factor NF-κB which regulates expression of various genes involved in immune response, and also the gene coding for the same and pharmacyeutical composition containing the same.

The inventors have proved that IKK-i is a novel serine/threonine kinase which phosphorylates IκB and activates NF-κB, and whose expression is induced by various inflammatory cytokines.

The present invention relates to a protein having an amino acid sequence represented by SEQ ID NO: 2 or SEQ ID NO: 4, or an amino acid sequence in which one or more amino acids in the said amino acid sequence is deleted or substituted by other amino acids and/or one or more other amino acid is added, and being able to activate the transcription factor NF-κB. The protein of the present invention is a novel serine/threonine kinase.

The present invention further relates to a gene having a base sequence which codes for the above mentioned novel protein. More particularly, the present invention pertains to a gene having a base sequence represented by SEQ ID NO: 1 or SEQ ID NO: 3.

The present invention more further relates to a pharmaceutical composition comprising the above mentioned protein and a pharmaceutically acceptable carrier thereof. The pharmaceutical composition of the present invention can activate the transcription factor NF-κB and also acts on the immune response mechanism. Further, the pharmaceutical composition of the present invention is useful as a preventive or therapeutic agent against diseases involving I-TRAF or TRAF molecule.

### Brief Description of Drawings

Fig. 1 is a drawing replaced by a photograph showing the results of northern blotting analysis, in which the induction of expression of mRNA of IKK-i before stimulation (-) or after stimulation (+) by LPS is shown. The lower photograph shows the results obtained using G3PDH.
Fig. 2 is a drawing replaced by a photograph showing the results of northern blotting analysis, in which expression of mRNA of IKK-i after LPS stimulation is shown in a time-dependent manner. The lower photograph shows the results obtained using G3PDH.
Fig. 3 shows a comparison of the amino acid sequences of human IKK-i and mouse IKK-i. In the figure, the part enclosed by a rectangle shows an identical sequence; a part [] shows a kinase domain; and * shows a leucine-zipper domain
Fig. 4 shows a comparison of the amino acid sequences of IKK-i, IKK-α and IKK-β. The parts colored with a gray backgrounds in the figure show identical sequences; the part [] shows a kinase domain; the part enclosed by a rectangle shows an activation loop; and * mark shows an amino acid residue which may be important for kinase activity.
Fig. 5 is a drawing replaced by a photograph showing the expression of IKK-i in various organs based on northern blotting analysis.
Fig. 6 is a drawing replaced by a photograph showing the expression of IKK-i in B cells and T cells based on northern blotting analysis. The lower photograph shows result of using G3PDH.
Fig. 7 is a drawing replaced by a photograph showing the expression of IKK-i in a mouse tumor strain based on northern blotting analysis. The lower photograph shows total RNA stained with ethidium bromide.
Fig. 8 shows a drawing replaced by a photograph showing the induction of IKK-i in mouse peritoneal macrophages as a result of various stimulations based on northern blotting analysis. The lower photograph shows the result obtained using G3PDH.
Fig. 9 shows the results of activation of the NF-κB reporter gene by enforced expression of IKK-i. The lower photograph is a drawing replaced by a photograph, showing the results of an evaluation of the amount of protein. These results were obtained by immunoblotting using anti-FLAG antibody (M2).
Fig. 10 is a drawing replaced by a photograph showing the results of phosphorylation of IκB-α by IKK-i in vitro. The lower photograph shows the results of evaluation of the amount of protein based on the results of immunoblotting using anti-FLAG antibody (M2).
Fig. 11 is a drawing replaced by a photograph showing the formation of complexes of IKK-i and I-TRAF in cells. In Fig. 11, lane 1 shows Flag-IKK-I, lane 2 shows Myc-I-TRAF and lane 3 shows Flag-IKK-i and Myc-I-TRAF respectively.
Fig. 12 is a drawing replaced by a photograph showing the binding region of IKK-i obtained by using an I-TRAF deletion mutant. In Fig. 12, lane 1 shows 1 - 170 fragment lane 2 shows 1 - 247 fragment, lane 3 shows 193 - stop fragment and lane 4 shows full length (FL) respectively.
Fig. 13 is a drawing replaced by a photograph showing the phosphorylation of I-TRAF by IKK-i. In lanes 1, 3 and 5 in Fig. 13, only Flag-IKK-i is transfected, and in lanes 2, 4 and 6, both of IKK-i and I-TRAF are transfected.
Fig. 14 is a drawing replaced by a photograph showing the phosphorylation region of I-TRAF. In Fig. 14, lanes 1 and 5 show 1 - 170 fragment, lanes 2 and 6 show 1 - 247 fragment, lanes 3 and 7 show 193 - stop fragment and lanes 4 and 8 show full length (FL) respectively.
Fig. 15 is a drawing replaced by a photograph showing the phosphorylation of purified GST-I-TRAF by IKK-i. In Fig. 15, lane 1 shows that Flag-IKK-i is transfected, and lane 2 shows that the mutant IKK-i (K38A) is transfected.

### Best Embodiment for Carrying Out the Invention

Firstly, cDNA cloning of IKK-i of the present invention is explained.

Using the suppression subtractive hybridization technique, a subtraction between a group with lipopolysaccharide (LPS) stimulation (+) and a group without LPS stimulation (-) was performed against RAW 264.7, a macrophage tumor strain. Subsequently, screening for the gene induced by LPS stimulation was carried out. Seven new gene fragments, in addition to known genes such as MIP-1α/β, G-CSF and TNF-α, were obtained.

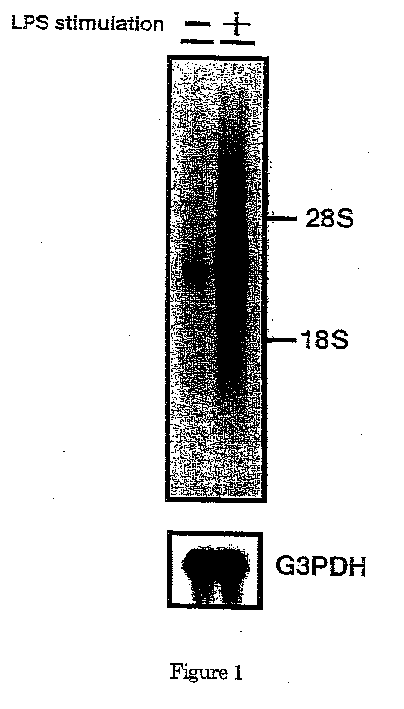

As the result of testing these fragments, only slight expression of clone #2F9 was observed in RAW 264.7 without stimulation, but the expression was markedly increased after 4 hours of the LPS stimulation (refer to Fig. 1). Fig. 1 shows the results of northern blotting analysis of the gene induced by lipopolysaccharide (LPS) stimulation of macrophage tumor strain RAW 264.7. Samples containing poly(A)⁺RNA 2µg before (-) or after (+) LPS (100 ng/ml) stimulation of RAW 264.7 were electrophoresed in 1% formamide-agarose and transferred to nylon membranes; they were then hybridized using a probe containing a cDNA fragment (2Fa) of IKK-i obtained by the subtraction. Fig. 1, lower photograph, shows that the amount of RNA is equivalent to the amount obtained by using G3PDH.

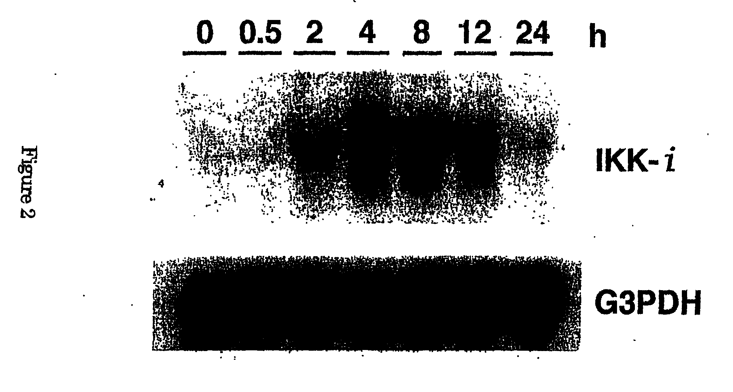

As for the time course of expression of the mRNA, it started to increase 2 hours after from LPS stimulation, reached a peak value after 4 hours, and returned to the original level after 24 hours (refer to Fig. 2).

Fig. 2 shows that the results based on northern blotting analysis are similar to those in Fig. 1. Namely, RAW 264.7 was stimulated by 100 ng/ml LPS, the total RNA was extracted after the indicated number of hours in each lane, then 25 µg of each sample was electrophoresed in 1% formamide-agarose. The-coding region of mouse IKK-i (mIKK-i) was used as a probe. In Fig. 2, the lower photograph shows that the amount of RNA is equivalent to that obtained by using G3PDH.

A cDNA library was prepared using mRNA obtained by LPS stimulation of RAW 264.7 for 4 hours, and the full length of this gene was obtained by using fragment 2F9 as a probe. This gene codes for 718 amino acids in a 2154 bp open reading frame. As a result of a homology search, a human cDNA clone KIAA 0151, for which the base sequence was determined but the function was not known, showed the highest homology in the database. The homology was 82.3% in the amino acid level, consequently 2F9 was thought to be a counterpart of KIAA 0515 in the mouse.

The gene showing the second highest homology next to KIAA 0151 was IκB kinase-α,β (IKK-α and IKK-β), which has been recently identified and demonstrated to phosphorylate IκB-α and to activate NF-κβ. The homology of the kinase domain is 29.1% and 30.1% in the amino acid level, respectively.

2F9 and KIAA have been confirmed to have a serine/threonine kinase domain in the N-terminal end, and a leucine-zipper domain in the center. Consequently, based on the similarities of the structures of IKK-α and IKK-β and the function of these molecules, for which more explanation will be given later, the inventors have named this novel kinase as inducible IKK (IKK-i).

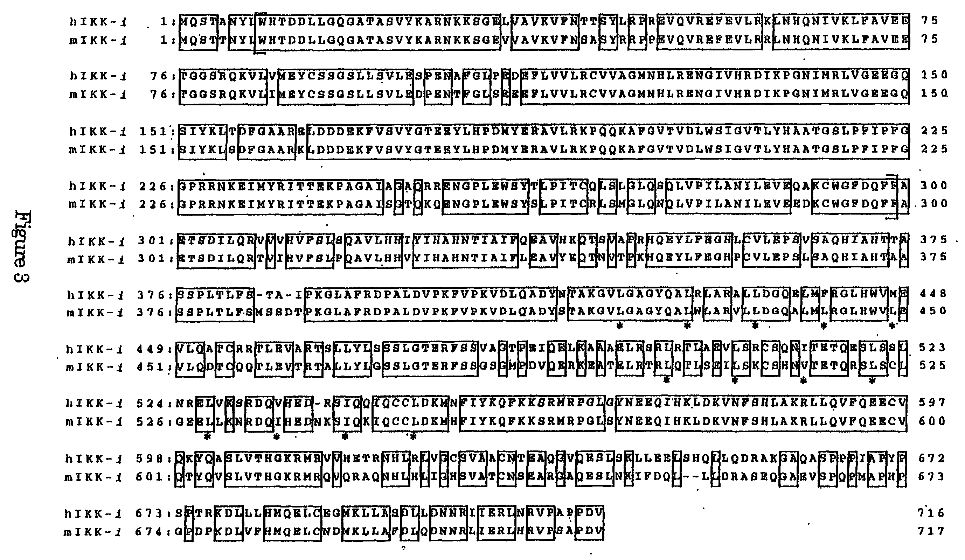

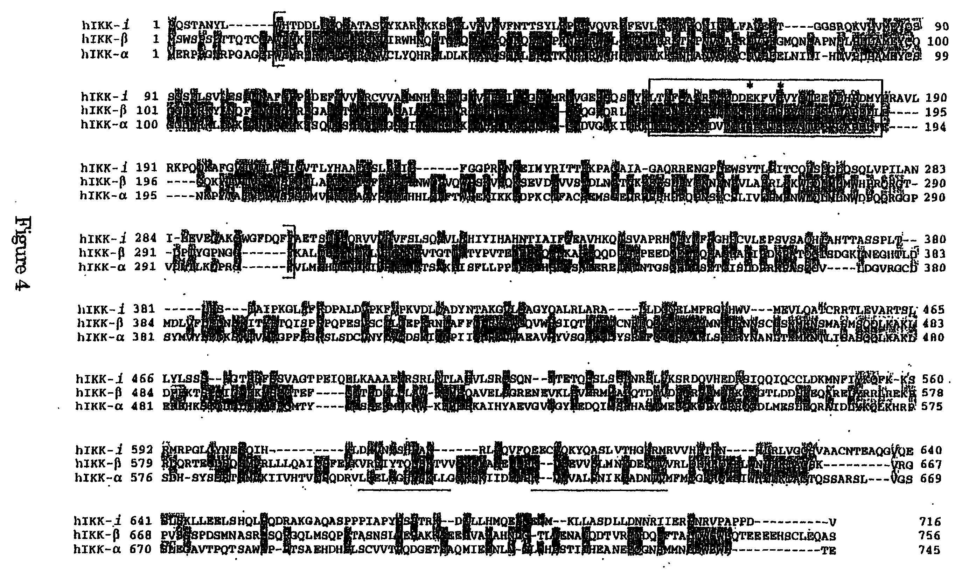

Comparisons of the amino acid sequences of human IKK-i and mouse IKK-i are shown in Fig. 3, and comparisons of the amino acid sequences of human IKK-i, human IKK-α and human IKK-β are shown in Fig. 4.

In Fig. 3, the identical sequences are enclosed by rectangles and the kinase domain is indicated by []. The leucine-zipper domain is indicated with an * mark below the domain.

In Fig. 4, the backgrounds of the identical sequences are colored with gray and the kinase domain is indicated by []. The activation loop is enclosed by a rectangle. The amino acid residues, which are thought to be important for kinase activity in the activation loop sequence, are indicated with an * mark. Under the helix loop - the helix structures of IKK-α and IKK-β are shown by underlining.

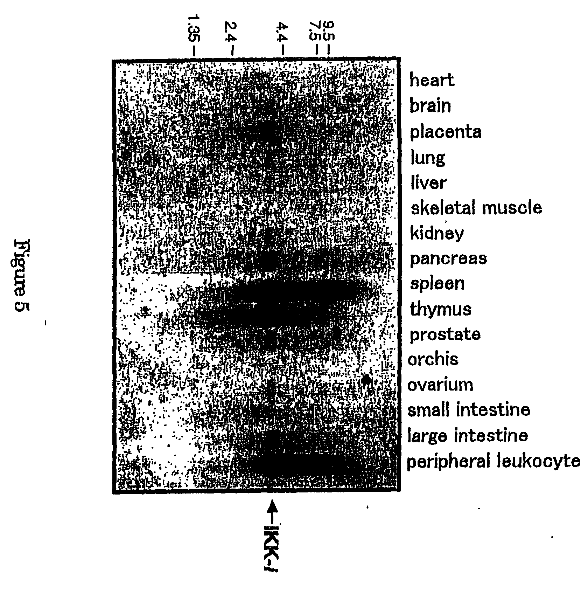

The expression of IKK-i in various organs was analyzed by northern blotting. IKK-α and IKK-β were generally expressed in all tissues, on the contrary, mRNA of IKK-i was primarily expressed specifically in spleen, thymus, peripheral leukocytes, pancreas and placenta (refer to Fig. 5). Northern blotting analysis was performed by using multi-tissue northern blot membranes (Clontech), in which 2 µg of poly (A)⁺RNA obtained from each of the indicated organs was placed in each lane shown in Fig. 5. A human IKK-i (hIKK-i) coding region was used as a probe.

In order to determine from which cell population the expression of IKK-i found in the spleen originated, B-cells and T-cells were isolated by using anti-B220 antibody. Before (-) and after (+) LPS stimulation of B-cells, and before (-) and after (+) phorbol ester and calcium ionophore stimulations of T-cells, the expression of IKK-i was analyzed by northern blotting. Though the expression was not able to be detected in B-cells without LPS stimulation, the expression was induced by LPS stimulation.

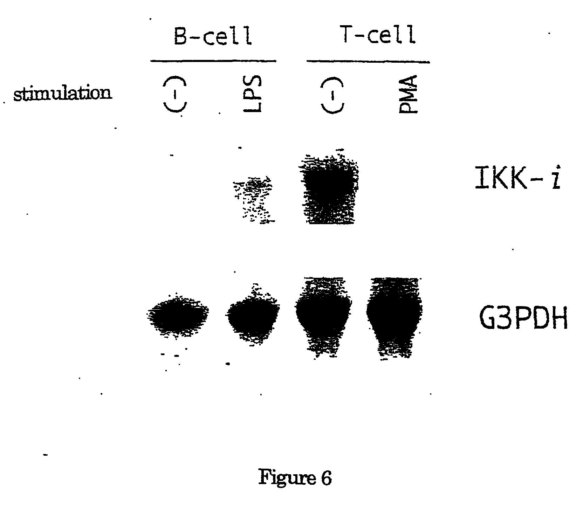

Constitative expression was observed in T-cells, but stimulation by phorbol ester and calcium ionophore reduced the expression (refer to Fig. 6).

Fig. 6 shows the results of northern blotting analysis carried out as follows. B-cells and T-cells were isolated from the spleen cells collected from C57BL/6 using antibody (B220) in a high-gradient magnetic cell separation system MACS (Miltenyi Biotec, Berg.-Gladbach, Germany). B-cells were stimulated by 100 µg/ml of LPS 100 and T-cells were stimulated by 10 µM of ionomycin and 10 µg/ml of PMA, each for 4 hours. Before and after the stimulation, the total RNA was extracted, and 20 µg of each sample was electrophoresed using 1% formamide agarose, and analyzed by northern blotting in the same way as the case in Fig. 2. The coding region of mouse IKK-i (mIKK-i) was used as a probe. In Fig. 6, the lower photograph shows that the amount of RNA obtained is equivalent to that obtained by using G3PDH.

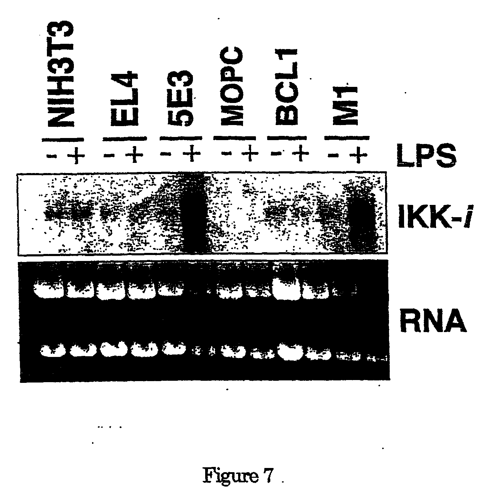

The expression of IKK-i in mouse cell strains was analyzed before (-) or after (+) stimulation. The expression of IKK-i was induced in 5E3 (a natural killer cell clone) and M1 (a monocytic leukemia cell line) by stimulation with LPS (refer to Fig. 7).

The total amounts of RNA before (-) and after (+) 4 hours of stimulation with LPS (100 ng/ml) were extracted from tumor strain NIH 3T3 (a fibroblast cell line), EL-4 (thymoma cells), 5E3 (a natural killer cell clone), MOPC 315 (myeloma cells), BCL-1 (B cell leukemia cells) and M1 (a monocytic leukemia cell line) shown in each lane in Fig. 7, and analyzed by northern blottings using the sane methods as shown in Fig. 2. The results are shown in Fig. 7. The coding region of mIKK-i was used as a probe. The lower part of Fig. 7 shows that the amount of RNA is equivalent to that obtained using the electrophoretic patterns of total RNA stained with ethidium bromide.

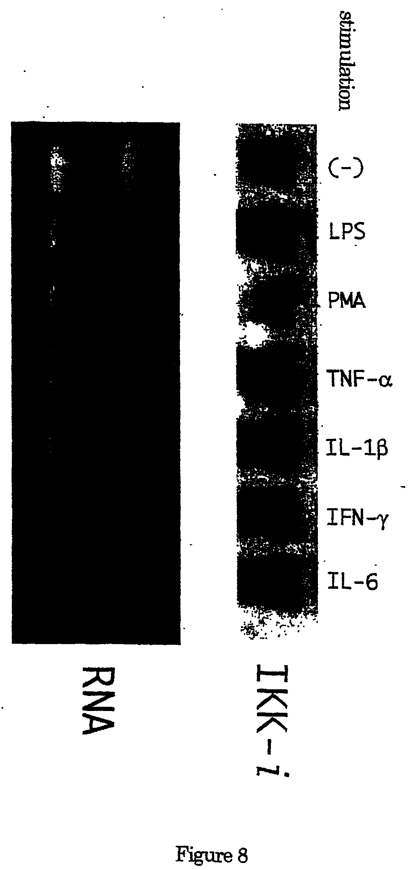

A further enhancement effect of stimulation, other than the effect of LPS on expression of IKK-i, was examined. Peritoneal macrophages collected from C57BL/6 were stimulated by LPS, PMA, TNF-α, IL-1β, IFN-γ or IL-6 for 4 hours, and the expression of IKK-i was observed. IKK-i was induced by TNF-α, IL-1β, IFN-γ or IL-6 instead of LPS, but was not induced by PMA (refer to Fig. 8).

Fig. 8 shows that the peritoneal macrophages collected from C57BL/6 were stimulated by LPS: 1 µg/ml, PMA: 10 ng/ml, RNF-α: 100 ng/ml, IL-1β: 100 ng/ml, IFN-γ: 250 U/ml or Il-6: 2000 U/ml as shown in the upper part of each lane in Fig. 8. Subsequently, the total RNA was extracted then analyzed by northern blottings in the same way as shown in Fig. 2. The coding region of mIKK-i was used as a probe. The lower part of Fig. 8 shows that the amount of RNA obtained is equivalent.

It has been proved by a reporter gene assay that enforced intracellular expression of IKK-α and IKK-β results in activating NF-κB. Owing to the structural similarities of IKK-i to them, the possibility of activating NF-κB might be expected. Consequently, the possibility of NF-κB activity of IKK-i was examined by a reporter gene assay.

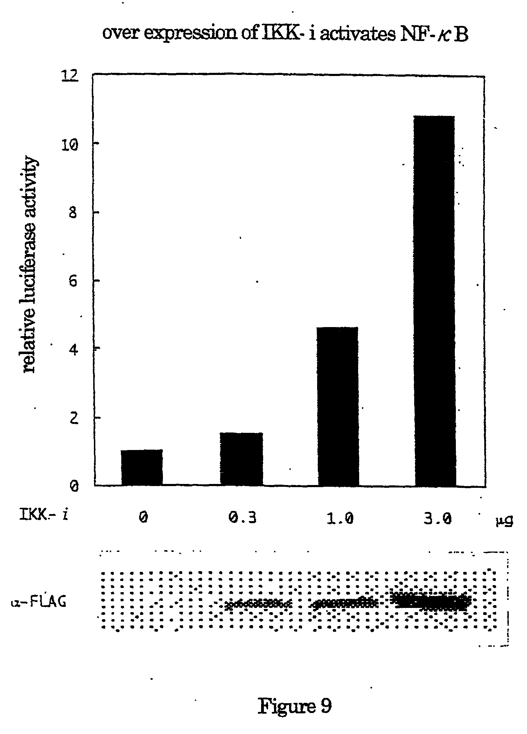

First, a construct (pEF-BOS-FLAG-WT-IKK-i), in which FLAG epitope was tagged onto the N-terminal of IKK-i and inserted into pEF-BOS expression vector, was prepared. Then, pEF-BOS-FLAG-IKK-i or control of a vector only were transiently cotransfected into the luciferase reporter construct of NF-κB and 293T cells, and luciferase activities were assayed. As the result, it was demonstrated that IKK-i activated NF-κB in an expression-dependent manner (refer to Fig. 9).

Fig.9 shows the results of the luciferase activity assay. In these studies, 293T cells were transiently cotransfected with the reporter construct (pNF-κB-Luc) in which luciferase gene was ligated with the NF-κB consensus sequence, the construct (pEF-BOS-FLAG-WT-IKK-i) in which FLAG epitope was tagged onto the N-terminal of IKK-i gene and subcloned into pEF-BOS expression vector, or a control of vector only. The total amounts of DNA were regularized to 4 µg by using a pEF-BOS vector. The mounts of transfected pEF-BOS-FLAG-WT-IKK-i are shown in the lower part of the graph, and the lower part of Fig. 9 shows the amount of protein, which was determined by immunoblotting using anti-FLAG antibody (M2).

IKK-α and IKK-β have been known to phosphorylate IκB-α in vitro. It was analyzed by an in vitro kinase assay whether or not the IKK-i of the present invention was able to phosphorylate the serine residues at No. 32 and No. 36.

A mutant construct (pEF-BOS-FLAG-K38A-IKK-i), which was prepared by mutating the No. 38 lysine of pEF-BOS-FLAG-WT-IKK-i or IKK-i to alanine, was transiently transfected into the 298T-cells. The IKK-i protein or the K38A-IKK-i protein expressed after 24 hours was purified by immunoprecipitation with anti-FLAG antibody (M2) and was used for an in vitro kinase assay.

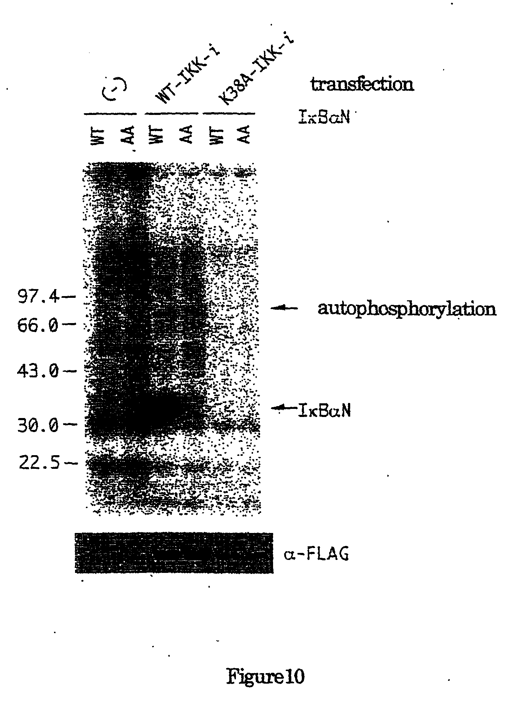

GST-IκB-αN protein (WT) which was prepared by removing the C-terminal from an ankyrin repeat of 1κB-α, or GST-IκB-αN protein (AA) which was prepared by replacing both of the No.32 and No.36 serine residues of GST-IκB-αN with alanine, was used as a substrate. A band of autophosphorylated IKK-i at about 80 kDa was observed in a lane of the BOS-FLAG-WT-IKK-i. Though IKK-i phosphorylated GST-I κB-αN protein (WT), K38A-IKK-i did not phosphorylate GST-IκB-α N protein (WT). Further, IKK-i did not phosphorylate GST-IκB-αN protein (AA)(refer to Fig. 10).

Fig. 10 shows the results of phosphorylation of IκB-α by IKK-i in vitro. A pEF-BOS-MOCK, a pEF-BOS-FLAG-WT-IKK-i, or a mutant construct (pEF-BOS-FLAG-K38A-IKK-I) which was prepared by mutating the No. 38 lysine of pEF-BOS-FLAG-WT-IKK-i to alanine, was transiently transfected into the 298T-cells. The IKK-i protein or the K38A-IKK-i protein expressed after 24 hours was purified by anti-FLAG antibody (M2) using immunoprecipitation, and was used for an in vitro kinase assay. GST-IκB-αN protein (WT) which was prepared by removing the C-terminal from an ankyrin repeat of IκB-α, or GST-IκB-αN protein (AA) which was prepared by replacing both of the No. 32 and No. 36 seine residues of GST-IκB-αN with alanine, was expressed in E. coli and purified using glutathione Sepharose, and the resulting products were used as substrates. IKK-i or K38A-IKK-i as substrates and [γ -³²P]ATP were reacted at 30°C for 20 minutes. The reaction mixture was developed with SDS-PAGE and measured by autoradiography. Arrows point to the bands of autophosphorylation and GST-IκB-αN. The molecular weight (kDa) is shown on the left side. The lower part of Fig. 10 shows the results of determination of the amount of protein by immunoblotting using anti-FLAG antibody (M2).

As a result, it was elucidated that the IKK-i of the present invention phosphorylated a serine residue in the N-terminal of IκB which played an important role in the activation of NF-κB.

The base sequences and amino acid sequences clarified as a result of cDNA cloning of the IKK-i of the present invention are shown in the sequence listings. SEQ ID NO: 1 shows the base sequence for human IKK-i (hIKK-i) and SEQ ID NO: 2 shows the amino acid sequence for hIKK-i. SEQ ID NO: 3 shows the base sequence for mouse IKK-i (mIKK-i) and SEQ ID NO: 4 shows the amino acid sequence for mIKK-i.

It was found that the IKK-i of the present invention was a novel serine/threonine kinase having a kinase domain in the N-terminal and a leucine-zipper domain in the center and the expression of its mRNA was induced by LPS stimulation of macrophages. The amino acid sequence of the IKK-i of the present invention showed high homologies with IKK-α and IKK-β, which phosphorylate Iκ B and activate NF-κB.

The IKK-i of the present invention was expressed constitutively in the spleen, thymus and peripheral leukocytes, and was also expressed constitutively in T-cells in the spleen. The expression was enhanced by LPS stimulation of B-cells, peritoneal macrophages, natural killer cells, and a monocyte tumor strain, and was also enhanced by stimulation of peritoneal macrophages by TNF-α, IL-1β, IFN-γ or IL-6. As a result of northern blotting analysis, it has been shown that expression of IKK-i is directed mainly to immune competent cells and cells involved in the inflammatory reaction, and IKK-i may be a molecule involved in the inflammatory reaction since it is enhanced by inflammatory stimulation.

Activation ability of IKK-i for NF-κB was analyzed by a reporter gene assay, and it was found that enforced expression of IKK-i in the 293T cells resulted in activating NF-κ B in a amount-of-protein-dependent manner. From the fact that IKK-i can phosphorylate a seine residue in the N-terminal of IκB-α in a manner similar to the actions of IKK-α and IKK-β, as elucidated by an in vitro kinase assay, it is suggested that the activation ability for the NF-κB observed in the reporter gene assay may be dependent on phosphorylation of the N-terminal of IκB-α by IKK-i. IKK-i is a novel IκB kinase, expression of which is induced by inflammatory stimulation of immune competent cells, and which activates NF-κB.

The IKK-i of the present invention may possibly have the ability to contribute to maintaining the activation of NF-κB by stimulation of the LPS. This is suggested by the facts that the amount of expression increases 2 hours after from LPS stimulation and NF-κB is activated in an expression-dependent manner. Since expression of IKK-i is directed to the immune competent cells as compared with IKK-α and IKK-β, the development of an inhibitor may possibly to be able to suppress the activation of NF-κB specific to the immune system. Consequently, controlling IKK-i may contribute to the treatment of inflammatory diseases.

Further, in order to isolate a molecule which interacts with the IKK-i of the present invention, a yeast two hybrid method was performed. Amino acids No. 541-716 from human IKK-i were inserted into a plasmid, pAS2-1, for expression of a chimera protein with a GAL4 DNA binding domain in order to prepare a bait plasmid. This was then transformed to yeast Y190 and grown on a selective medium. The transformant thus obtained was further transformed with a pACT2 plasmid containing a human B-cell derived cDNA library which was able to express the chimera protein with a GAL4 activation domain. Cells were then grown on a selective medium. The plasmid was recovered from the positive clones thus obtained. Finally the base sequence was determined by using a DNA sequencer. As a result of a homology search, 10 clones were found to be identical to the sequence I-TRAF/TANK which had been already known.

It was examined that IKK-i and I-TRAF were able to be bound to each other in cells. First, an expression vector, which was able to express in mammalian cells, was constructed. Flag was linked to the N-terminal of human IKK-i as an epitope, and then inserted into the expression vector pEF-BOS. Myc was added to the N-terminal of human I-TRAF as an epitope, and then inserted into the expression vector pEF-BOS. These were both transfected into a monkey kidney cell line, COS-7 cells, by lipofection. After 24 hours, the cells were solubilized with buffer containing 1.0% Nonidet P-40. The resulting material was immunoprecipitated by anti-Flag antibody or anti-Myc antibody, and was then analyzed by means of western blotting using anti-Myc antibody or anti-Flag antibody.

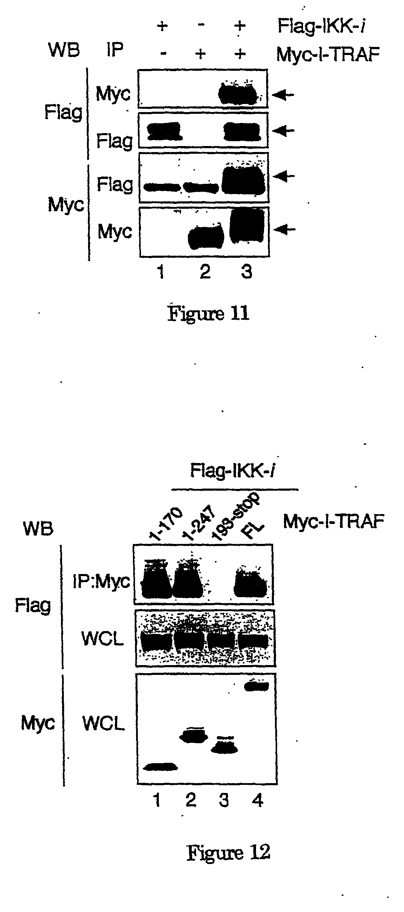

Results are shown in Fig. 11. In Fig. 11, lane 1 shows Flag-IKK-I, lane 2 shows Myc-I-TRAF, lane 3 shows Flag-IKK-i and Myc-I-TRAF respectively. Bands including Flag-IKK-I which was immunoprecipitated with anti-Myc antibody (Fig. 11, upper part, lane 3) and Myc-I-TRAF which was coimmunoprecipitated with anti-Flag antibody (Fig. 11, lower middle parts, lane 3) were specifically detected. Consequently, both molecules were demonstrated to form complexes in the mammalian cells. The amount of expression in each lane can be confirmed by means of western blotting analysis of the immunoprecipitates of anti-Myc antibody or anti-Flag antibody with anti-Myc antibody or anti-Flag antibody (Fig. 11, upper middle and lower parts).

Next, it was determined through which domain of the I-TRAF IKK-i is bound. This was examined by using the same methods as the above. First, three types of I-TRAF defective mutants were prepared. A fragment of the amino acid sequence of amino acids No. 1 - 170, 1 - 247 or 193 - C-terminal (stop) from human I-TRAF to which Myc was added to the N-terminal thereof, was inserted into pEF-BOS. These were then transfected with Flag-IKK-i into COS-7 cells. After 24 hours the cells were solubilized. The resulting solubilized materials were immunoprecipitated with anti-Myc antibody, then analyzed by means of western blotting with anti-Flag antibody.

The results are shown in Fig. 12. In Fig. 12, lane 1 shows 1 - 170 fragment, lane 2 shows 1 - 247 fragment, lane 3 shows 193 - stop fragment and lane 4 shows FL (full length) respectively. In the expressed cells, a band of Flag-IKK-i, which is immunoprecipitated by anti-Myc antibody, is detected (Fig. 12, upper part). Consequently, it was demonstrated that I-TRAF bound with IKK-i through the 170 amino acids in the N-terminal. The amount of expressed protein in each lane was confirmed by means of western blotting analysis using anti-Flag antibody (Fig. 12, middle part) or anti-Myc antibody (Fig. 12, lower part) with the material described above.

Next, It was determined whether or not I-TRAF is a substrate fur phosphorylation by IKK-i. Flag-IKK-i and Myc-I-TRAF were transfected into COS-7 cells and solubilized after 24 hours. After immunoprecipitating the resulting material with anti-Flag antibody or anti-Myc antibody, kinase buffer and [γ -³²P]ATP were added to the precipitate and allowed to react. Then an in vitro kinase assay was performed.

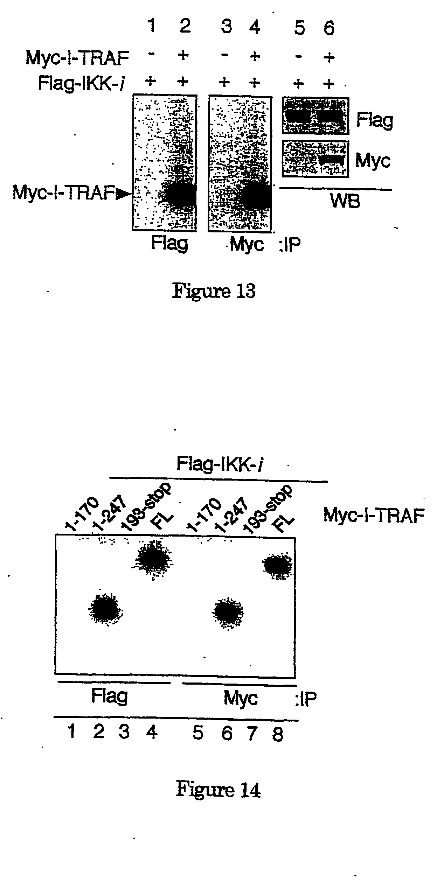

The results are shown in Fig. 13. Lanes 1, 3 and 5 show the results of transfection with only Flag-IKK-i, and lanes 2, 4 and 6 show the results of transfection with both Flag-IKK-i and Myc-I-TRAF. Bands of phosphorylated Myc-I-TRAF were detected in both of immunoprecipitates with anti-Flag antibody (lane 2) or anti-Myc antibody (lane 4). Consequently, it was clearly demonstrated that Myc-I-TRAF was a substrate which was phosphorylated by IKK-i. The amounts of expression of protein in each lane were confirmed by means of western blotting analysis of the products of solubilizing with anti-Flag antibody (Fig. 13, upper parts of lanes 5 and 6) or anti-Myc antibody (Fig. 13, lower parts of lanes 5 and 6).

Next, it was examined which region of I-TRAF was required for phosphorylation. Myc-I-TRAF defective mutants [a fragment of amino acids No. 1 - 170, a fragment of amino acids No. 1 - 247, a fragment of amino acids No. 197 - stop and full length (FL)] were transfected with IKK-i into COS-7 cells and the resulting solubilized material was obtained. After that, immunoprecipitation was performed with anti-Flag antibody or anti-Myc antibody and an in vitro assay was performed.

The results are shown in Fig. 14. In Fig. 14, lanes 1 and 5 show a fragment of amino acids No. 1 - 170, lanes 2 and 6 show a fragment of amino acids No. 1 - 247, lanes 3 and 7 show a fragment of amino acids No. 197 - stop and lanes 4 and 8 show full length (FL) respectively. As shown, phosphorylations of FL (lanes 4 and 8) and Myc-I-TRAF (1-247)(lanes 2 and 6) were confirmed. Consequently, the region, where the phosphorylation by IKK-I occurs, may be located at least within amino acids No. 171 - 247 in the amino acid sequence of I-TRAF.

Further, it was examined whether or not I-TRAF was phosphorylated by IKK-i by using the purified protein of I-TRAF. First, the I-TRAF protein was purified. Human I-TRAF cDNA was then inserted into an expression vector pGEX-5X-i, which was able to be expressed as a chimera protein with glutathione S transferase (GST) in E. coli. The vector thus obtained was transformed into E. coli DH5α. After E. coli was cultured overnight in LB liquid medium, IPTG was added and the mixture was incubated for 3 hours. The collected E. coli cells were solubilized by PBS and disrupted by ultrasonication. Triton X-100 was added up to 1% to solubilize the mixture, glutathione Sepharose was added and the mixture was allowed to react for 1 hour. GST-I-TRAF protein bound with glutathione Sepharose was eluted with a glutathione solution and used as the purified protein for the experiment. Flag-IKK-i was transfected into COS-7 cells. Simultaneously, a mutant IKK-i (K38A) was prepared and transfected. In the K38A mutant, the No. 38 lysine in the kinase domain, which is thought to be an ATP binding site, is substituted by alanine. It is known that kinase activity is lost by a mutation at this site in many kinases. After 24 hours of transfection, cells were solubilized and immunoprecipitated with anti-Flag antibody. A purified GST-I-TRAF (1.0 µg) was added to the immunoprecipitate and phosphorylated in vitro to perform a kinase assay.

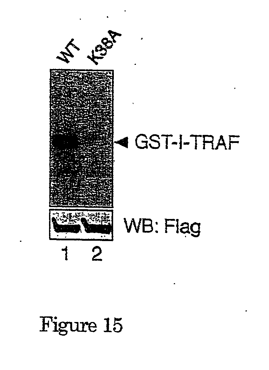

The results are shown in Fig. 15. In Fig. 15, lane 1 shows the results of transfection with Flag-IKK-i and lane 2 shows results of transfection with mutant IKK-i (K38A). As shown, in cells in which Flag-IKK-i was expressed, phosphorylation of GST-I-TRAF was observed (Fig. 15, upper part of lane 1). No phosphorylation was observed with the K38A mutant (Fig. 15, upper part of lane 2). Consequently, it was elucidated that I-TRAF was a substrate which was phosphorylated by IKK-i. Further, intracellular expression of the K38A was confirmed by means of western blotting analysis of the solubilized product with anti-flag antibody (Fig. 15, lower part).

As a result, the binding of IKK-i with I-TRAF was elucidated. Further it was shown by an in vitro kinase assay that I-TRAF was a specific substrate which was phosphorylated by IKK-i. I-TRAF was firstly identified as a molecule bound with TRAF 2 and TRAF 3. At present, six types of TRAF molecules have been identified and are known to function as adapter molecules bound with various receptors. Especially, the TRAF molecule binds with an apoptosis-related TNF receptor and CD40, and has been known as a signal transduction molecule for these receptors.

The TRAF molecule is activated by forming a complex with a receptor as a result of stimulation by a ligand. However, when activation does not occur, activation is thought to be negatively regulated by binding with I-TRAF in the cytoplasm. Consequently, IKK-i is thought to be involved indirectly in the activation of the TRAF molecule by phosphorylation of the I-TRAF.

As previously explained, since the IKK-i of the present invention is involved in activation of the TRAF molecule, which relates to apoptosis, it is useful for the prevention, treatment and regulation of apoptosis-related diseases related to the TRAF molecule.

Also as previously explained, IKK-i is thought to be a very interesting molecule from a clinical standpoint. A pharmaceutical composition of the present invention is comprised of IKK-i and pharmaceutically acceptable carrier and can be administered in the form for administration. The dosage can be adjusted according to the situation of the patients.

The pharmaceutical composition of the present invention is effective for improvement of the immune response function and for treatments for inflammatory diseases.

The present invention also includes the use of antisense against the IKK-i gene as a component of the pharmaceutical composition of the present invention.

### Examples

Following examples illustrate the present invention but are not construed as limiting the invention.

### Example 1 (cDNA cloning of mIKK-i)

Using the suppression subtractive hybridization technique, a subtraction between a group with 100 ng/ml of lipopolysaccharide (LPS) stimulation (+) and a group without the LPS stimulation (-) was performed against. RAW 264.7, a macrophage tumor strain. Subsequently, screening for the gene induced by LPS stimulation was carried out. As a result, seven new gene fragments, in addition to known genes such as MIP-1α/β, G-CSF and TNF-α, were obtained. Among seven novel genes, clone #2F9 was a gene fragment having 374 bp. In order to obtain full length of gene of the clone #2F9, a cDNA library was prepared using λZAP phage from mRNA obtained by LPS stimulation of RAW 264.7 for 4 hours. A fragment of 374 bp in the 2F9 was labeled with α-³²P-dCTP by random labeling technique. A full length gene was obtained by screening of cDNA phage library using the labeled 2F9 as a probe.

The full length of the clone thus obtained was 2910 bp. This gene codes for 718 amino acids in a 2154 bp open reading frame. The base sequence of this gene is shown in SEQ ID NO: 3. The amino acid sequence is shown in SEQ ID NO: 4.

### Example 2 (cDNA cloning of hIKK-i)

Based on the previously registered base sequence KIAA0151 in DDBJ, cDNA of human IKK-i was cloned by means of PCR using human placental cDNA library as a template. The sequences of the primer used for PCR were as follows:
5' -ctttgcctgactcagggcagctcagag-3', and
5' -atggtgcagaagagcagtgttggaatc-3'

This gene coded 716 amino acids in 2148 bp open reading frame. The base sequence of this gene is shown in SEQ ID NO:1. Amino acid sequence is also shown in SEQ ID NO: 2.

### Example 3 (Expression of clone #2F9 by LPS stimulation)

The northern blotting analysis of the gene induced by lipopolysaccharide (LPS) stimulation of macrophage tumor strain RAW 264.7 was performed. Samples containing 2 µg of poly(A)⁺RNA before (-) or after (+) LPS (100 ng/ml) stimulation of RAW 264.7 were electrophoresed in 1% formamide-agarose and transferred to nylon membranes, then hybridized using a probe containing a cDNA fragment (2Fa) of IKK-i obtained by the subtraction.

Results are shown in Fig. 1. The lower photograph in Fig.1 shows that the amount of RNA is equivalent to the amount obtained by using G3PDH.

### Example 4 (Time-course of expression of done #2F9 by LPS stimulation)

RAW 264.7 was stimulated by 100 ng/ml of LPS, the total RNA was extracted after 0.5, 2, 4, 8, 12 and 24 hours, respectively, then 25 µg of each sample was electrophoresed in 1% formamide-agarose and northern blotting analysis was performed similar to those in example 3. The coding region of mouse IKK-i (mIKK-i) obtained in example 2 was used as a probe.

The results are shown in Fig. 2. The lower photograph in Fig. 2 shows that the amount of RNA is equivalent to that obtained by using G3PDH.

### Example 5 (Expression of IKK-i)

The expression of IKK-i in various organs was analyzed by northern blotting.

Northern blotting analysis was performed by using multi-tissue northern blot membranes (Clontech), in which 2 µg of poly (A)⁺RNA obtained from each organ was placed. A human IKK-i (hIKK-i) coding region was used as a probe.

Results are shown in Fig. 5. In Fig. 5, an arrow indicates a position of IKK-i.

### Example 6 (Expression of IKK-i in spleen)

B-cells and T-cells were isolated from the spleen cells collected from C57BL/6 using antibody (B220) in a high-gradient magnetic cell separation system MACS (Miltenyi Biotec, Berg.-Gladbach, Germany). B-cells were stimulated by 100 µg/ml of LBS and T-cells were stimulated by 10 µM of ionomycin and 10 µg/ml of PMA, each for 4 hours. Before and after the stimulation, the total RNA was extracted, and 20 µg each of the samples were electrophoresed using 1% formamide agarose, and analyzed by northern blotting in the same way as the case in example 4. The coding region of mIKK-i was used as a probe.

Results are shown in Fig. 6. In Fig. 6, the lower photograph shows that the amount of RNA obtained is equivalent to that obtained by using G3PDH.

### Example 7 (Expression of IKK-i in cells of mice)

The total amounts of RNA before (-) and after (+) 4 hours of the stimulation with LPS (100 ng/ml) were extracted from tumor strain NIH 3T3 (a fibroblast cell line), EL-4 (thymoma cells), 5E3 (a natural killer cell clone), MOPC 315 (myeloma cells), BCL-1 (B cell leukemia cells) and M1 (a monocytic leukemia cell line) of mouse cell strains, and analyzed by northern blottings using the same methods as shown in example 4. The coding region of mIKK-i was used as a probe.

The results are shown in Fig. 7. The lower part of Fig. 7 shows that the amount of RNA is equivalent to that obtained using the electrophoretic patterns of total RNA stained with ethidium bromide.

### Example 8 (Expression of IKK-i in peritoneal macrophages)

The peritoneal macrophages collected from C57BL/6 were stimulated by LPS: 1 µg/ml, PMA: 10 ng/ml, RNF-α: 100 ng/ml, IL-1β: 100 ng/ml, IFN-γ: 250 U/ml or Il-6: 2000 U/ml. Subsequently, the total RNA was extracted then analyzed by northern blottings in the same way as in example 4. The coding region of mIKK-i was used as a probe.

Results are shown in Fig. 8. In Fig. 8, (-) indicates a case without stimulation.

The lower part of Fig. 8 shows that the amount of RNA obtained is equivalent to that obtained by using G3PPH.

### Example 9 (Construction of IKK-i expression vector)

FLAG epitope was tagged to the N-terminal end of IKK-i gene. Restriction enzyme SalI sites were constructed in the 5'- and 3'-ends of FLAG-hIKK-i fragment using primer sequences (1) and (2) of the following formulae by applying with PCR.
( 1 )5' -gggtcgacca ccatggacta caaggacgac gatgacaaga tgcagagcac agccaat-3'
( 2 )5' -gtcgactcag accatcagga ggtgc-3'

The resulted sequence was subcloned into T-vector (p-GEM-T)(Promega), excised by using the restriction enzyme SalI, and subcloned into pEF-BOS expression vector to construct the expression vector pEF-BOS-FLAG-WT-IKK-i.

### Example 10 (Activation of NF-κB by IKK-i)

The NF-κB consensus reporter construct (pNF-κB-Luc)(Stratagene Inc.) and IKK-i expression vector, 0 µg (without addition), 0.3 µg, 1.0 µg and 3.0 µg of pEF-BOS-FLAG-WT-IKK-i obtained in example 9 were added respectively to 3 × 10⁵ cells of 293T cells, and transiently cotransfected by lipofection using Than it LT-1 (Pan Vera Corp.). Then luciferase activity was assayed by using Dual Luciferase Reporter assay system (Promega Inc.). Vector only was added for the control group. The total amount of DNA was regularized to 4 µg by using pEF-BOS vector.

Results are shown in Fig. 9. The Lower part of Fig. 9 shows the amount of protein, which was determined by immunoblotting using anti-FLAG antibody (M2).

### Example 11 (Preparation of a mutant of IKK-i, in which No. 38 lysine is replaced by alanine)

The bases coding No. 38 lysine in IKK-i were replaced by the bases coding alanine and a gene of IKK-i mutant was prepared by means of point mutation technique. Transformer site directed mutagenesis kit (Clontech Inc.) was used for point mutation.

### Example 12 (Construction of expression vector of mutant of IKK-i)

The expression vector of the mutant, pEF-BOS-FLAG-K38A-IKK-i, was prepared by the same way as of in example 9 using the mutant gene obtained in example 11.

### Example 13 [Preparation of GST-IκB-αN protein (WT)]

A gene coding amino acids 1 - 72 in the amino acid sequence, in which the C-terminal part was split from ankyrin repeat in IκB-α, was prepared and this was expressed in E. coli. The resulted product was purified by using glutathione Sepharose to obtain GST-IκB-αN protein (WT). PGEX2T (Pharmacia Inc.), was used as the vector.

### Example 14 [Preparation of GST-IκB-αN protein (AA)]

A gene, in which both of bases coding for the No. 32 and No. 36 serine residues of GST-IκB-αN were replaced by the bases coding for alanine, was prepared. This was expressed in E. coli and purified using glutathione Sepharose to obtain GST-IκB-αN protein (AA).

### Example 15 (Phosphorylation of serine residue of IκB-α by IKK-i)

The pEF-BOS-MOCK, the pEF-BOS-FLAG-WT-IKK-i obtained in example 9, or the mutant construct (pEF-BOS-FLAG-K38A-IKK-i) obtained in example 12, was transiently transfected to 2 × 10⁶ cells of 293T-cells on 10 cm dishes. After 24 hours, expressed IKK-i protein or K-38A-IKK-i protein was purified by means of immunoprecipitation and measured by in vitro kinase assay.

The GST-IκB-αN protein (WT) obtained in example 14 or the protein (AA) obtained in example 15 was used as a substrate for the in vitro kinase assay.

IKK-i or K38A-IKK-i, substrates and [γ -³²P]ATP were reacted at 30°C for 20 minutes. The reaction mixture was developed with SDS-PAGE and measured by autoradiography.

The results are shown in Fig. 10. In Fig. 10, the arrow pointing to a position of about 80 kDa shows a band of autophosphorylation and the arrow below it shows a band of GST-IκB-αN. The molecular weight (kDa) is shown on the left side in Fig. 10. The lower part of Fig. 10 shows the results of determination of the amount of protein by immunoblotting using anti-FLAG antibody (M2).

As a results, K38A-IKK-i did not phosphorylate GST-IκB-αN protein (WT) though IKK-i phosphorylated GST-IκB-αN protein (WT). Further, IKK-i did not phosphorylate GST-IκB-αN protein (AA).

### Example 16 (Isolation of the molecule which interacts with IKK-i using yeast two hybridization)

Amino acids No. 541 - 716 from human IKK-i were inserted into a plasmid, pAS2-1, for expression of a chimera protein with a GAL4 DNA binding domain in order to prepare a bait plasmid. This was then transformed to yeast Y190 and grown on a selective medium. The transformant thus obtained was further transformed with a pACT2 plasmid containing a human B-cell derived cDNA library which was able to express the chimera protein with a GAL4 activation domain. The cells were then grown on a selective medium. The plasmid was recovered from the positive clones thus obtained. Finally the base sequence was determined by using a DNA sequencer. As a result of a homology search, 10 clones were found to be identical to the sequence 1-TRAF/TANK which had been already known.

### Example 17 (Binding with IKK-i and I-TRAF in cells)

Flag was linked to the N-terminal of human IKK-i as an epitope, and then inserted into the expression vector pEF-BOS.

Myc was added to the N-terminal of human I-TRAF as an epitope, and then inserted into the expression vector pEF-BOS.

Further, the Flag was linked to the N-terminal of human IKK-i as an epitope, and a sequence, in which Myc was added to the N-terminal of human I-TRAF as an epitope, was added thereto, then inserted into the expression vector pEF-BOS.

These were transfected into a monkey kidney cell line, COS-7 cells, by lipofection. After 24 hours, the cells were solubilized with buffer containing 1.0% Nonidet P-40. The resulting solubilized material was immunoprecipitated by anti-Flag antibody or anti-Myc antibody, and was then analyzed by means of western blotting using anti-Myc antibody or anti-Flag antibody.

Results are shown in Fig. 11. In Fig. 11, lane 1 shows Flag-IKK-I, lane 2 shows Myc-I-TRAF, lane 3 shows Flag-IKK-i and Myc-I-TRAF respectively.

### Example 18 (Determination of binding region of I-TRAF and IKK-i)

First three types of I-TRAF defective mutants consisting of a fragment of the amino acid sequence, amino acids No. 1 - 170, 1 - 247 or 193 - C-terminal (stop) of human I-TRAF, to which Myc was added to the N-terminal thereof, were prepared. These were inserted into pEF-BOS respectively, then transfected with Flag-IKK-i into COS-7 cells. The cells were solubilized after 24 hours, and the resulting solubilized materials were immunoprecipitated with anti-Myc antibody, then analyzed by means of western blotting with anti-Flag antibody.

The results are shown in Fig. 12. In Fig. 12, lane 1 shows 1 - 170 fragment, lane 2 shows 1 - 247 fragment, lane 3 shows 193 - stop fragment and lane 4 shows FL (full length) respectively.

### Example 19 (Confirmation of phosphorylation of I-TRAF by IKK-i)

Flag-IKK-i and Myc-I-TRAF were transfected into COS-7 cells and solubilized after 24 hours. After immunoprecipitating the resulting solubilized material with anti-flag antibody or anti-Myc antibody, kinase buffer and [γ-³²P]ATP were added to the precipitate and allowed to react. Then an in vitro kinase assay was performed.

The results are shown in Fig. 13. Lanes 1, 3 and 5 show the results of transfection with Flag-IKK-I only, and lanes 2, 4 and 6 show the results of transfection with both Flag-IKK-i and Myc-I-TRAF. As shown in Fig. 13, bands of phosphorylated Myc-I-TRAF were detected in evey immunoprecipitates with anti-Flag antibody (lane 2) or anti-Myc antibody (lane 4). Consequently, it was clearly demonstrated that Myc-I-TRAF was a substrate which was phosphorylated by IKK-i. The amounts of expression of protein in each lane were confirmed by means of western blotting analysis of the products of solubilizing with anti-Flag antibody (upper part of lanes 5 and 6) or anti-Myc antibody (lower part of lanes 5 and 6).

### Example 20 (Determination of phosphorylated region in I-TRAF)

Myc-I-TRAF defective mutants [1 - 170, 1 - 247, 197 - stop and full length (FL)] were transfected with IKK-i into COS-7 cells and the resulting solubilized material was obtained. After that, immunoprecipitation was performed with anti-Flag antibody or anti-Myc antibody and an in vitro assay was performed.

The results are shown in Fig. 14. In Fig. 14, lanes 1 and 5 show a fragment of amino acids No. 1 - 170, lanes 2 and 6 show a fragment of amino acids No. 1 - 247, lanes 3 and 7 show a fragment of amino acids No. 197 - stop and lanes 4 and 8 show full length (FL) respectively.

### Example 21 (Phosphorylation of I-TRAF protein by IKK-i)

Human I-TRAF cDNA was then inserted into an expression vector pGEX-5X-i, which was able to be expressed as a chimera protein with glutathione S transferase (GST) in E. coli. The vector thus obtained was transformed into E. coli DH5α. After E. coli was cultured overnight in LB liquid medium, IPTG was added and the mixture was incubated for 3 hours. The collected E. coli cells were solubilized by PBS and disrupted by ultrasonication. Triton X-100 was added up to 1% to solubilize the mixture, glutathione Sepharose was added and the mixture was allowed to react for 1 hour. GST-I-TRAF protein bound with glutathione Sepharose was eluted with a glutathione solution and used as the purified protein for the experiment.

Next, Flag-IKK-i was transfected into COS-7 cells. Simultaneously, a mutant IKK-i (K38A) was prepared and transfected.

After 24 hours of transfection, the cells were solubilized and immunoprecipitated with anti-flag antibody. A purified GST-I-TRAF (1.0 µg) was added to the immunoprecipitate and phosphorylated in vitro to perform a kinase assay.

The results are shown in Fig. 15. In the cells in which Flag-IKK-i was expressed, phosphorylation of GST-I-TRAF was observed (Fig. 15, upper part of lane 1). No phosphorylation was observed with the K38A mutant (Fig. 15, upper part of lane 2). Further, intracellular expression of the K38A was confirmed by means of western blotting analysis of the product of solubilizing with anti-Flag antibody (Fig. 15, lower part).

### Industrial Applicability

The present invention provides a novel IκB kinase, IKK-i, which is a novel serine/threonin kinase capable of activating transcription factor NF-κB which regulates expression of various genes involved in immune response. The present invention also provides the gene coding for the same and a pharmaceutical composition containing the same. Since IKK-i of the present invention phosphorylates IκB and activates NF-κB, it is effective for improvement of the immune response function and for treatments for inflammatory diseases to regulate this gene. Further, IKK-i of the present invention binds with I-TRAF to phosphorylate the same, and is effective for regulation of TRAF molecule activity involved in apoptosis.

SEQUENCE LISTING

<110> Japan Science And Technology Corporation

<120> Identification of Novel Substrate I-TRAF of IKK-i Kinase

<130> JA901491

<160> 4

<210> 1
<211> 2154
<212> DNA
<213> Human
<400> 1
agatgcagag cacagccaat tacctgtggc acacagatga cctgctgggg caggggggcca 60
ctgccagtgt gtacaaggcc cgcaacaaga aatccggaga gctggttgct gtgaaggtct 120
tcaacactac cagctacctg cggccccgcg aggtgcaggt gagggagttt gaggtcctgc 180
ggaagctgaa ccaccagaac atcgtcaagc tctttgcggt ggaggagacg ggcggaagcc 240
ggcagaaggt actggtgatg gagtactgct ccagtgggag cctgctgagt gtgctggaga 300
gccctgagaa tgcctttggg ctgcctgagg atgagttcct ggtggtgctg cgctgtgtgg 360
tggccggcat gaaccacctg cgggagaacg gcattgtgca tcgcgacatc aagccgggga 420
acatcatgcg cctcgtaggg gaggaggggc agagcatcta caagctgaca gacttcggcg 480
ctgcccggga gctggatgat gatgagaagt tcgtctcggt ctatgggact gaggagtacc 540
tgcatcccga catgtatgag cgggcggtgc ttcgaaagcc ccagcaaaaa gcgttcgggg 600
tgactgtgga tctctggagc attggagtga ccttgtacca tgcagccact ggcagcctgc 660
ccttcatccc ctttggtggg ccacggcgga acaaggagat catgtaccgg atcaccacag 720

agaagccggc tggggccatt gcaggtgccc agaggcggga gaacgggccc ctggagtgga 780
gctacaccct ccccatcacc tgccagctgt cactggggct gcagagccag ctggtgccca 840
tcctggccaa catcctggag gtggagcagg ccaagtgctg gggcttcgac cagttctttg 900
cggagaccag tgacatcctg cagcgagttg tcgtccatgt cttctccctg tcccaggcag 960
tcctgcacca catctatatc catgcccaca acacgatagc cattttccag gaggccgtgc 1020
acaagcagac cagtgtggcc ccccgacacc aggagtacct ctttgagggt cacctctgtg 1080
tcctcgagcc cagcgtctca gcacagcaca tcgcccacac gacggcaagc agccccctga 1140
ccctcttcag cacagccatc cctaaggggc tggccttcag ggaccctgct ctggacgtcc 1200
ccaagttcgt ccccaaagtg gacctgcagg cggattacaa cactgccaag ggcgtgttgg 1260
gcgccggcta ccaggccctg cggctggcac gggccctgct ggatgggcag gagctaatgt 1320
ttcgggggct gcactgggtc atggaggtgc tccaggccac atgcagacgg actctggaag 1380
tggcaaggac atccctcctc tacctcagca gcagcctggg aactgagagg ttcagcagcg 1440
tggctggaac gcctgagatc caggaactga aggcggctgc agaactgagg tccaggctgc 1500
ggactctagc ggaggtcctc tccagatgct cccaaaatat cacggagacc caggagagcc 1560
tgagcagcct gaaccgggag ctggtgaaga gccgggatca ggtacatgag gacagaagca 1620
tccagcagat tcagtgctgt ttggacaaga tgaacttcat ctacaaacag ttcaagaagt 1680
ctaggatgag gccagggctt ggctacaacg aggagcagat tcacaagctg gataaggtga 1740
atttcagtca tttagccaaa agactcctgc aggtgttcca ggaggagtgc gtgcagaagt 1800
atcaagcgtc cttagtcaca cacggcaaga ggatgagggt ggtgcacgag accaggaacc 1860
acctgcgcct ggttggctgt tctgtggctg cctgtaacac agaagcccag ggggtccagg 1920
agagtctcag caagctcctg gaagagctat ctcaccagct ccttcaggac cgagcaaagg 1980
gggctcaggc ctcgccgcct cccatagctc cttaccccag ccctacacga aaggacctgc 2040
ttctccacat gcaagagctc tgcgagggga tgaagctgct ggcatctgac ctcctggaca 2100
acaaccgcat catcgaacgg ctaaatagag tcccagcacc tcctgatgtc tgag 2154

<210> 2
<211> 716
<212> PRT
<213> Human

<400> 2

Met Gln Ser Thr Ala Asn Tyr Leu Trp His Thr Asp Asp Leu Leu 15

Gly Gln Gly Ala Thr Ala Ser Val Tyr Lys Ala Arg Asn Lys Lys 30

Ser Gly Glu Leu Val Ala Val Lys Val Phe Asn Thr Thr Ser Tyr 45

Leu Arg Pro Arg Glu Val Gln Val Arg Glu Phe Glu Val Leu Arg 60

Lys Leu Asn His Gln Asn Ile Val Lys Leu Phe Ala Val Glu Glu 75

Thr Gly Gly Ser Arg Gln Lys Val Leu Val Met Glu Tyr Cys Ser 90

Ser Gly Ser Leu Leu Ser Val Leu Glu Ser Pro Glu Asn Ala Phe 105

Gly Leu Pro Glu Asp Glu Phe Leu Val Val Leu Arg Cys Val Val 120

Ala Gly Met Asn His Leu Arg Glu Asn Gly Ile Val His Arg Asp 135

Ile Lys Pro Gly Asn Ile Met Arg Leu Val Gly Glu Glu Gly Gln 150

Ser Ile Tyr Lys Leu Thr Asp Phe Gly Ala Ala Arg Glu Leu Asp 165

Asp Asp Glu Lys Phe Val Ser Val Tyr Gly Thr Glu Glu Tyr Leu 180

His Pro Asp Met Tyr Glu Arg Ala Val Leu Arg Lys Pro Gln Gln 195

Lys Ala Phe Gly Val Thr Val Asp Leu Trp Ser Ile Gly Val Thr 210

Leu Tyr His Ala Ala Thr Gly Ser Leu Pro Phe Ile Pro Phe Gly 225

Gly Pro Arg Arg Asn Lys Glu Ile Met Tyr Arg Ile Thr Thr Glu 240

Lys Pro Ala Gly Ala Ile Ala Gly Ala Gln Arg Arg Glu Asn Gly 255

Pro Leu Glu Trp Ser Tyr Thr Leu Pro Ile Thr Cys Gln Leu Ser 270

Leu Gly Leu Gln Ser Gln Leu Val Pro Ile Leu Ala Asn Ile Leu 285

Glu Val Glu Gln Ala Lys Cys Trp Gly Phe Asp Gln Phe Phe Ala 300

Glu Thr Ser Asp Ile Leu Gln Arg Val Val Val His Val Phe Ser 315

Leu Ser Gln Ala Val Leu His His Ile Tyr Ile His Ala His Asn 330

Thr Ile Ala Ile Phe Gln Glu Ala Val His Lys Gln Thr Ser Val 345

Ala Pro Arg His Gln Glu Tyr Leu Phe Glu Gly His Leu Cys Val 360

Leu Glu Pro Ser Val Ser Ala Gln His Ile Ala His Thr Thr Ala 375

Ser Ser Pro Leu Thr Leu Phe Ser Thr Ala Ile Pro Lys Gly Leu 390

Ala Phe Arg Asp Pro Ala Leu Asp Val Pro Lys Phe Val Pro Lys 405

Val Asp Leu Gln Ala Asp Tyr Asn Thr Ala Lys Gly Val Leu Gly 420

Ala Gly Tyr Gln Ala Leu Arg Leu Ala Arg Ala Leu Leu Asp Gly 435
Gln Glu Leu Met Phe Arg Gly Leu His Trp Val Met Glu Val Leu 450
Gln Ala Thr Cys Arg Arg Thr Leu Glu Val Ala Arg Thr Ser Leu 465
Leu Tyr Leu Ser Ser Ser Leu Gly Thr Glu Arg Phe Ser Ser Val 480
Ala Gly Thr Pro Glu Ile Gln Glu Leu Lys Ala Ala Ala Glu Leu 495
Arg Ser Arg Leu Arg Thr Leu Ala Glu Val Leu Ser Arg Cys Ser 510
Gln Asn Ile Thr Glu Thr Gln Glu Ser Leu Ser Ser Leu Asn Arg 525
Glu Leu Val Lys Ser Arg Asp Gln Val His Glu Asp Arg Ser Ile 540
Gln Gln Ile Gln Cys Cys Leu Asp Lys Met Asn Phe Ile Tyr Lys 555
Gln Phe Lys Lys Ser Arg Met Arg Pro Gly Leu Gly Tyr Asn Glu 570
Glu Gln Ile His Lys Leu Asp Lys Val Asn Phe Ser His Leu Ala 585
Lys Arg Leu Leu Gln Val Phe Gln Glu Glu Cys Val Gln Lys Tyr 600
Gln Ala Ser Leu Val Thr His Gly Lys Arg Met Arg Val Val His 615
Glu Thr Arg Asn His Leu Arg Leu Val Gly Cys Ser Val Ala Ala 630
Cys Asn Thr Glu Ala Gln Gly Val Gln Glu Ser Leu Ser Lys Leu 645
Leu Glu Glu Leu Ser His Gln Leu Leu Gln Asp Arg Ala Lys Gly 660
Ala Gln Ala Ser Pro Pro Pro Ile Ala Pro Tyr Pro Ser Pro Thr 675
Arg Lys Asp Leu Leu Leu His Met Gln Glu Leu Cys Glu Gly Met 690
Lys Leu Leu Ala Ser Asp Leu Leu Asp Asn Asn Arg Ile Ile Glu 705
Arg Leu Asn Arg Val Pro Ala Pro Pro Asp Val *** 716

<210> 3
<211> 2910
<212> DNA
<213> Mouse
<400> 3
gaattcggca cgagaagata gccaagccca ggagatgcag agtaccacta actacctgtg 60
gcatactgat gacctgctag ggcagggggc cactgccagt gtgtacaagg cccgaaacaa 120
gaaatccggg gaggtggttg ctgtaaaggt cttcaactca gccagctatc ggcgacctcc 180

tgaggttcag gtgagggagt ttgaggtcct gcggaggctg aatcaccaga acatcgtgaa 240
gctattcgca gtggaggaaa cgggaggcag ccggcagaag gtgctaatca tggagtactg 300
ctccagtggg agcctgctga gcgtgctgga agaccctgag aacacgttcg ggctttctga 360
agaggagttc ctagtggtgc tgcgctgtgt ggtggctggc atgaaccacc tgcgggagaa 420
tggcattgtc catcgggaca tcaaacctgg gaacatcatg cgcctggtgg gcgaggaggg 480
gcagagcatc tataagctgt ctgacttcgg ggctgcccgc aagctggacg atgatgagaa 540
gtttgtttct gtctatggta cagaggaata cctgcaccct gacatgtatg agcgtgcagt 600
gctgcgcaaa ccccagcaaa aggcatttgg tgtgactgtg gatctctgga gtattggggt 660
gaccctgtac cacgcagcca caggcagtct gcccttcatc cccttcggtg ggccccggcg 720
caacaaagag atcatgtaca gaatcaccac agagaagcca gccggggcca tttcaggcac 780
tcagaagcag gaaaatggtc ccttggagtg gagctacagc ctccccatca cctgtagact 840
gtccatgggg ctgcagaacc agctggtgcc catcctggcc aacatcctgg aggtggaaga 900
ggataagtgc tggggctttg atcagttctt cgcggagacc agtgacattc tgcagcgaac 960
ggtcatccac gtcttttccc taccccaggc cgttttgcat catgtctaca tccacgccca 1020
caacacgatt gccatctttt tggaggctgt atatgagcag accaacgtga cccccaaaca 1080
ccaggagtac ctcttcgagg gtcacccttg tgtccttgag ccaagcctct cagcccagca 1140
catcgcccac acagctgcca gcagccctct aactctgttc agcatgtcca gcgacacacc 1200
taaggggctg gccttcaggg accctgctct ggatgtccca aagttcgtcc ctaaggttga 1260
cctacaggcc gattacagca cagctaaggg ggtgctgggc gctggctacc aggccctgtg 1320
gctggcgcgg gtcctgctgg atggacaggc gttgatgctt cgggggttac attgggtcct 1380
ggaggtgctt caggacacgt gccagcagac actggaggtc acacggacag ccctcctcta 1440
cctcggcagc agcctgggca ctgaaaggtt cagcagtgga tcggggatgc ctgacgtcca 1500
ggaacgaaag gaggccacag agctaagaac caggctgcag actctctcag agatcctgtc 1560
taaatgttcc cacaatgtca cagaaaccca aaggagcctg agctgtctgg gtgaagagct 1620
tttaaagaac cgggaccaga ttcatgagga taacaaaagt atccagaaga ttcagtgttg 1680
tttggacaag atgcacttca tctacaaaca gttcaagaaa tccaggatga ggccagggct 1740
cagctacaat gaggagcaga tccacaagct ggataaggta aatttcagtc atctagccaa 1800
gaggctgctg caggtgttcc aggaggagtg tgtgcagacg tatcaggtgt cgctggtcac 1860
acacggcaag cggatgaggc aggtgcagag ggcccagaac cacctgcatc tcattggcca 1920

ctctgtggcc acctgtaact cggaagcccg gggagcccag gagagtctga acaagatctt 1980
tgatcagctc cttctggaca gagcttccga acagggagct gaggtgtcac cgcaacctat 2040
ggctcctcat cccggccctg atccgaagga cctggtcttc cacatgcagg agctttgtaa 2100
tgatatgaag ctattggcct ttgatctcca ggacaacaac cgactcatcg aacggttaca 2160
tagagttcca tcggcaccag atgtctgagc tccctggggg ttcacaaggc actcagaagc 2220
aatagaaaca ttcatattgt acccctacac tgtgagacca aattcagggc aagttctggt 2280
tccatctcac tagcctacct ccctcttggc cattggccat tggccaacaa actagcatta 2340
ctttgactgt cctcttggga agcagctagg acagggactc ctggccatcc caggcagtat 2400
ctacagaaga gaccatgcgg ctaccacagc cttatcaaga caccaagact gttcttcctt 2460
acccaggctc tggaggtctg gtcttggaaa gaaaaggctc agccctctca cgctttgcac 2520
ttcccaggac cagcaggcat ctcctgtggc ttctcctgcc tctccagggt gctggatcag 2580
aatgcttatt cttcgttgtt tcctgtgctg tttcctgagt gtccccatcc cctggcctca 2640
ggcaacccac aaacggcccc tctgtgcttg gtctagatgc acctgcattt gagaaagtgg 2700
gtggttgagg ctaactgctg gtgctttgag gattctcctt gaccttttct ccgaggaacg 2760
cttggttcta agaaacagct ggtcagtatc aaccacagcc atgctaactg gacagatgtt 2820
ggaacccaaa gtcctaagga gagagcaggc ctgcaccttc agacatggaa taaatacatc 2880
gccttttctg tttaaaaaaa aaaaaaaaaa 2910

<210> 4
<211> 717
<212> PRT
<213> Mouse
Met Gln Ser Thr Thr Asn Tyr Leu Trp His Thr Asp Asp Leu Leu 15
Gly Gln Gly Ala Thr Ala Ser Val Tyr Lys Ala Arg Asn Lys Lys 30
Ser Gly Glu Val Val Ala Val Lys Val Phe Asn Ser Ala Ser Tyr 45
Arg Arg Pro Pro Glu Val Gln Val Arg Glu Phe Glu Val Leu Arg 60
Arg Leu Asn His Gln Asn Ile Val Lys Leu Phe Ala Val Glu Glu 75
Thr Gly Gly Ser Arg Gln Lys Val Leu Ile Met Glu Tyr Cys Ser 90
Ser Gly Ser Leu Leu Ser Val Leu Glu Asp Pro Glu Asn Thr Phe 105

Gly Leu Ser Glu Glu Glu Phe Leu Val Val Leu Arg Cys Val Val 120
Ala Gly Met Asn His Leu Arg Glu Asn Gly Ile Val His Arg Asp 135
Ile Lys Pro Gly Asn Ile Met Arg Leu Val Gly Glu Glu Gly Gln 150
Ser Ile Tyr Lys Leu Ser Asp Phe Gly Ala Ala Arg Lys Leu Asp 165
Asp Asp Glu Lys Phe Val Ser Val Tyr Gly Thr Glu Glu Tyr Leu 180
His Pro Asp Met Tyr Glu Arg Ala Val Leu Arg Lys Pro Gln Gln 195
Lys Ala Phe Gly Val Thr Val Asp Leu Trp Ser Ile Gly Val Thr 210
Leu Tyr His Ala Ala Thr Gly Ser Leu Pro Phe Ile Pro Phe Gly 225
Gly Pro Arg Arg Asn Lys Glu Ile Met Tyr Arg Ile Thr Thr Glu 240
Lys Pro Ala Gly Ala Ile Ser Gly Thr Gln Lys Gln Glu Asn Gly 255
Pro Leu Glu Trp Ser Tyr Ser Leu Pro Ile Thr Cys Arg Leu Ser 270
Met Gly Leu Gln Asn Gln Leu Val Pro Ile Leu Ala Asn Ile Leu 285
Glu Val Glu Glu Asp Lys Cys Trp Gly Phe Asp Gln Phe Phe Ala 300
Glu Thr Ser Asp Ile Leu Gln Arg Thr Val Ile His Val Phe Ser 315
Leu Pro Gln Ala Val Leu His His Val Tyr Ile His Ala His Asn 330
Thr Ile Ala Ile Phe Leu Glu Ala Val Tyr Glu Gln Thr Asn Val 345
Thr Pro Lys His Gln Glu Tyr Leu Phe Glu Gly His Pro Cys Val 360
Leu Glu Pro Ser Leu Ser Ala Gln His Ile Ala His Thr Ala Ala 375
Ser Ser Pro Leu Thr Leu Phe Ser Met Ser Ser Asp Thr Pro Lys 390
Gly Leu Ala Phe Arg Asp Pro Ala Leu Asp Val Pro Lys Phe Val 405
Pro Lys Val Asp Leu Gln Ala Asp Tyr Ser Thr Ala Lys Gly Val 420
Leu Gly Ala Gly Tyr Gln Ala Leu Trp Leu Ala Arg Val Leu Leu 435
Asp Gly Gln Ala Leu Met Leu Arg Gly Leu His Trp Val Leu Glu 450
Val Leu Gln Asp Thr Cys Gln Gln Thr Leu Glu Val Thr Arg Thr 465
Ala Leu Leu Tyr Leu Gly Ser Ser Leu Gly Thr Glu Arg Phe Ser 480
Ser Gly Ser Gly Met Pro Asp Val Gln Glu Arg Lys Glu Ala Thr 495
Glu Leu Arg Thr Arg Leu Gln Thr Leu Ser Glu Ile Leu Ser Lys 510
Cys Ser His Asn Val Thr Glu Thr Gln Arg Ser Leu Ser Cys Leu 525
Gly Glu Glu Leu Leu Lys Asn Arg Asp Gln Ile His Glu Asp Asn 540

Lys Ser Ile Gln Lys Ile Gln Cys Cys Leu Asp Lys Met His Phe 555
Ile Tyr Lys Gln Phe Lys Lys Ser Arg Met Arg Pro Gly Leu Ser 570
Tyr Asn Glu Glu Gln Ile His Lys Leu Asp Lys Val Asn Phe Ser 585
His Leu Ala Lys Arg Leu Leu Gln Val Phe Gln Glu Glu Cys Val 600
Gln Thr Tyr Gln Val Ser Leu Val Thr His Gly Lys Arg Met Arg 615
Gln Val Gln Arg Ala Gln Asn His Leu His Leu Ile Gly His Ser 630
Val Ala Thr Cys Asn Ser Glu Ala Arg Gly Ala Gln Glu Ser Leu 645
Asn Lys Ile Phe Asp Gln Leu Leu Leu Asp Arg Ala Ser Glu Gln 660
Gly Ala Glu Val Ser Pro Gln Pro Met Ala Pro His Pro Gly Pro 675
Asp Pro Lys Asp Leu Val Phe His Met Gln Glu Leu Cys Asn Asp 690
Met Lys Leu Leu Ala Phe Asp Leu Gln Asp Asn Asn Arg Leu Ile 705
Glu Arg Leu His Arg Val Pro Ser Ala Pro Asp Val *** 717

## Claims

1. A protein comprising an amino acid sequence represented by SEQ ID NO: 2 or SEQ ID NO: 4, or an amino acid sequence, in which one or more amino acids in the said amino acid sequence are deleted or replaced by other amino acids, and/or one or more other amino acids are added, and capable of activating transcription factor NF-κB.

2. The protein according to claim 1 which is serine/threonine kinase.

3. The protein according to claims 1 or 3 which has activities for binding with I-TRAF and for phosphorylating I-TRAF.

4. A gene comprising base sequence coding the protein according to claim 1.

5. The gene according to claim 4, wherein the base sequence comprises the base sequence represented by SEQ ID NO: 1 or SEQ ID NO: 3.

6. A pharmaceutical composition comprising the protein according to any one of claims 1 - 3 and a pharmaceutically acceptable carrier.

7. The pharmaceutical composition according to claim 6, which acts on the immune response mechanism.

8. The pharmaceutical composition according to claim 6, which is a preventive or therapeutic agent for diseases involving the I-TRAF or the TRAF molecule.
